# EUROPÄISCHE PATENTANMELDUNG

(11) **EP 1 256 565 A2**
(43) Veröffentlichungstag der Anmeldung: **13.11.2002**
(21) Anmeldenummer: 02010048.3
(22) Anmeldetag: 06.05.2002
(51) Int. Cl.: C07C 67/03, C07C 69/52, C07C 69/24, C11C 3/04

(54) **Verfahren zur Herstellung von Fettsäureestern unter Einwirkung eines elektromagnetischen Feldes**

(30) Priorität: 07.05.2001 DE 10122011
(71) Anmelder: Hochschule Zittau/Görlitz (FH), 02763 Zittau (DE)
(72) Erfinder: Die Erfindernennung liegt noch nicht vor

(57) **Zusammenfassung**

Die Erfindung betrifft ein Verfahren zur Herstellung von langkettigen Fettsäureestern einwertiger Alkohole, wobei die an sich bekannten Umsetzungen in diskontinuierlicher oder kontinuierlicher Reaktionsführung unter Einwirkung eines elektromagnetischen Feldes durchgeführt werden.

Fettsäureester einwertiger Alkohole finden in der chemischen und pharmazeutischen Industrie sowohl als Rohstoff als auch als Zwischenprodukte vielfältige Verwendung. Darüber hinaus kommen derartige Verbindungen auch in der Nahrungsmittelindustrie und insbesondere als Kraftstoff für Dieselmotoren zum Einsatz.

Der vorliegenden Erfindung liegt die Aufgabe zugrunde, ein möglichst einfaches und wirtschaftliches Verfahren zur Herstellung von Fettsäureestern einwertiger Alkohole bereitzustellen, das möglichst geringe Anforderungen an die Ausgangsverbindungen stellt, in sehr kurzen Reaktionszeiten hohe Ausbeuten garantiert und sowohl als Batch- als auch als kontinuierliches Verfahren betrieben werden kann.

Diese Aufgabe wird erfindungsgemäß dadurch gelöst, dass die Umesterung natürlich vorkommender oder synthetischer Öle und/oder Fette in Fettsäureester einwertiger Alkohole basen- oder säurekatalysiert unter Einwirkung eines elektromagnetischen Feldes stattfindet.

## Beschreibung

Die Erfindung betrifft ein Verfahren zur Herstellung von langkettigen Fettsäureestern einwertiger Alkohole, wobei die an sich bekannten Umsetzungen in diskontinuierlicher oder kontinuierlicher Reaktionsführung unter Einwirkung eines elektromagnetischen Feldes durchgeführt werden.
Fettsäureester einwertiger Alkohole finden in der chemischen und pharmazeutischen Industrie sowohl als Rohstoff als auch als Zwischenprodukte vielfältige Verwendung. Darüber hinaus kommen derartige Verbindungen auch in der Nahrungsmittelindustrie und insbesondere als Kraftstoff für Dieselmotoren zum Einsatz.
Zur Herstellung dieser Verbindungen sind verschiedene Wege ausgehend von Erdöl oder nachwachsenden Rohstoffen vorgeschlagen worden. Eine besondere Bedeutung kommt der Darstellung solcher Ester ausgehend von pflanzlichen und/oder tierischen Ölen und Fetten zu.
Die bisherigen Verfahren zur Herstellung von Fettsäureestern basieren auf der basenkatalysierten Umesterung von Fettsäuren mehrwertiger Alkohole, insbesondere von Fettsäuretriglyceriden. Stellvertretend für das umfangreiche Schrifttum zu diesem Gebiet seien die Veröffentlichungen in J. Am. Oil Chem. Soc. **61** (1984) 384 und Ullmann, Enzyklopädie der Technischen Chemie, 4. Auflage, Band 11, Seite 432 ff. genannt.
Die bekannten technischen Verfahren zur Herstellung von Fettsäureestern einwertiger Alkohole durch basenkatalysierte Umesterung von Fettsäureestern mehrwertiger Alkohole (DE 39 32 514, DE 42 09 779, EP 0 562 504, EP 0 523 767) arbeiten bei hoher Temperatur und /oder hohem Druck und schließen aufwendige Aufarbeitungsschritte wie Destillationen ein. Damit benötigen diese Verfahren einen hohen technisch-apparativen und energetischen Aufwand und arbeiten somit entsprechend kostenintensiv. Aus diesen Gründen lassen sich derartige Verfahren wirtschaftlich nur im großtechnischen Maßstab ab etwa 100 000 Jahrestonnen durchführen. Für kleinere Anlagen eignen sich diese Verfahren nicht.
Es existieren Verfahren (DE 199 25 871, AT 386 222, AT 397 966, AT 387 399, DE 37 27 981, DE 30 20 612, DE 31 07 318, WO 92/00268), in denen Möglichkeiten aufgezeigt werden, Fettsäureester einwertiger Alkohole durch basenkatalysierte Umesterung von Fettsäuretriglyceriden wirtschaftlich im Bereich von 500 bis 5000 Jahrestonnen herzustellen. Nachteilig bei diesem Verfahren sind vor allem die langen Reaktionszeiten bis zur Produktbildung und die fehlende Möglichkeit, die Verfahren kontinuierlich zu betreiben.

Der vorliegenden Erfindung liegt daher die Aufgabe zugrunde, ein möglichst einfaches und wirtschaftliches Verfahren zur Herstellung von Fettsäureestern einwertiger Alkohole bereitzustellen, das möglichst geringe Anforderungen an die Ausgangsverbindungen stellt, in sehr kurzen Reaktionszeiten hohe Ausbeuten garantiert und sowohl als Batch- als auch als kontinuierliches Verfahren betrieben werden kann.

Diese Aufgabe wird erfindungsgemäß dadurch gelöst, dass die Umesterung natürlich vorkommender oder synthetischer Öle und/oder Fette in Fettsäureester einwertiger Alkohole basen- oder säurekatalysiert unter Einwirkung eines elektromagnetischen Feldes gemäß nachfolgend dargestelltem Schema stattfindet.

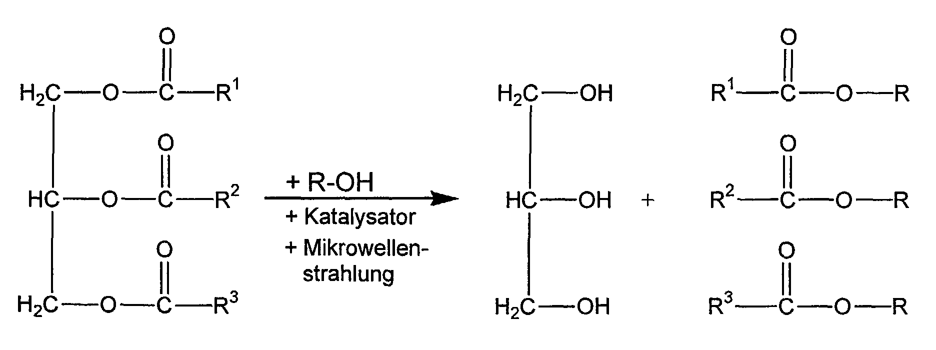

Dabei sind R¹ bis R³ Alkyl-, Alkenyl- und Alk(polyen)yl-Reste, R-OH stellt primäre, sekundäre und tertiäre Alkohole von C₁ bis C₄₀ dar und Katalysator bedeutet die Verwendung saurer oder basischer Katalysatoren bei dieser Umesterungsreaktion.

Die Reaktion wird vorzugsweise so durchgeführt, dass Reaktionsgemisch in einer Apparatur drucklos oder unter Druck bei Kontrolle von Energieeintrag, Druck und Temperatur der elektromagnetischen Strahlung ausgesetzt wird. Dabei ist der Reaktor so aufgebaut, dass sowohl diskontinuierliche als auch kontinuierliche Reaktionsführung möglich ist. Nach einer sehr kurzen Reaktionszeit arbeitet man mit den üblichen Methoden auf. Der Umsatz bei dieser Art der Reaktionsführung ist quantitativ, die Reaktionsprodukte fallen mit einer Reinheit von > 98.5 % an.

Die folgenden Beispiele sollen den Gegenstand der Erfindung näher erläutern.

### Ausführungsbeispiele

1. Eine Mischung aus 50 g (0,056 mol) Rapsöl, 10 g (0,31 mol) Methanol und 5,2 g Katalysatorlösung (0,2 g Natriumhydroxid oder Kaliumhydroxid in 5 g Methanol gelöst) wird für fünf Minuten in Gegenwart von Hochfrequenzstrahlung (2,45 GHz) unter Rückfluß erwärmt. Dabei wird bis zum Siedebeginn (nach etwa 0,5 min) mit 300 Watt bestrahlt, in der Folgezeit mit 150-200 W. Nach der Reaktion wird die untere glycerinhaltige Schicht abgetrennt und die obere Schicht, die die Methylester enthält, nach üblichen Methoden aufgearbeitet. Die Ausbeute beträgt 96% der Theorie bezogen auf cis-octadec-9-ensäure- und cis-docos-9-ensäurehaltiges Rapsöl.
2. Ein Gemisch aus 8,84 g (0,01 mol) Rapsöl und 7,8 g (0.06 mol) 1-Octanol werden 15 Minuten in Gegenwart von 0,5 ml H₂SO₄ in Gegenwart von Hochfrequenzstrahlung (2,45 GHz) unter Rückfluß erwärmt. Während der ganzen Zeit beträgt die Leistung 200 W. Nach Reaktionsende wird die das Produkt enthaltende Phase abgetrennt, gewaschen, getrocknet und im Vakuum destilliert. Ausbeute: 87% der Theorie bezogen auf Rapsöl.
3. Eine Mischung aus 3 Liter Rapsöl, 768 ml Methanol und 468 ml methylalkoholischer Natriumhydroxydlösung (siehe Bsp.1) werden durch einen Rohrreaktor (Innenvolumen 13 ml) mit einem Volumenstrom von 30 l/h einer Hochfrequenzstrahlung (2,45 GHz) ausgesetzt. Die zugeführte Leistung beträgt über die gesamte Reaktionszeit 500 W. Die Ausbeute beträgt 94 % bezogen auf cis-octadec-9-ensäurehaltiges Rapsöl.
4. In einem Glasdruckreaktor (3 bar) wird eine Mischung von 8,84 g Rapsöl (0,1 mol), 27,6 g (0,6 mol) Ethanol und 0,5 ml konzentrierter H₂SO₄ (96 %) unter Einwirkung von Hochfrequenzstrahlung (2,45 GHz) erwärmt. Die Leistung über die gesamte Reaktionszeit von 15 Minuten beträgt 100 W. Danach wird nach üblichen Methoden aufgearbeitet. Die Ausbeute beträgt 84 %.

## Patentansprüche

1. Verfahren zur Herstellung von Fettsäureestern unter Einwirkung eines elektromagnetischen Feldes ausgehend von natürlich vorkommenden oder synthetischen Ölen und/oder Fetten, **gekennzeichnet dadurch dass**
• nach dem allgemeinen Schema 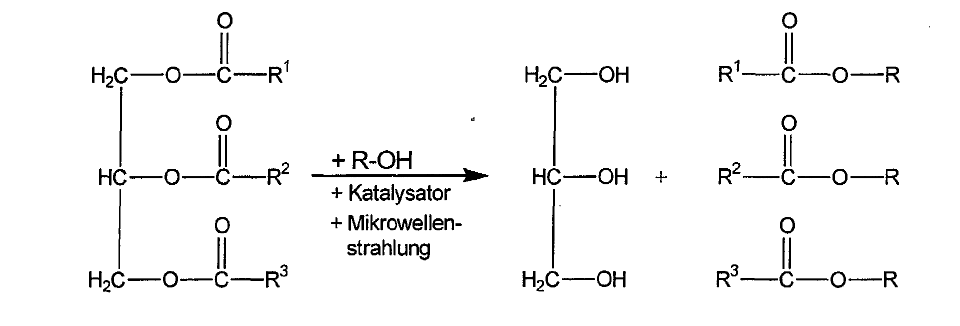
• worin üblicherweise R¹, R² und R³ = Alkyl, Alkenyl, Alk(polyen)yl, R = Alkyl, sec-Alkyl, tert.-Alkyl mit Kettenlängen von C₁ bis C₄₀, Cycloalkyl sowie Aryl sind,
• die Reaktionen in einem elektromagnetischen Feld im Frequenzbereich zwischen 10 MHz und 23 GHz
• als Umesterungsreaktion
• unter Einsatz saurer oder basischer Katalysatoren
• in Abhängigkeit vom Zielprodukt drucklos oder unter Druck bis 200 bar,
• in Abhängigkeit vom Zielprodukt in einem Temperaturbereich von 50 bis 250 °C
durchgeführt werden.

2. Verfahren nach Anspruch 1, **gekennzeichnet dadurch, dass** vorzugsweise bei 2,45 GHz gearbeitet wird.

3. Verfahren nach den Ansprüchen 1 und 2, **gekennzeichnet dadurch, dass** die Umesterungsreaktion als Batchverfahren abläuft.

4. Verfahren nach den Ansprüchen 1 und 2, **gekennzeichnet dadurch, dass** die Umesterungsreaktion kontinuierlich abläuft.
